# EUROPÄISCHE PATENTSCHRIFT

(11) **EP 1 249 231 B1**
(45) Veröffentlichungstag und Bekanntmachung des Hinweises auf die Patenterteilung: **28.01.2004**
(21) Anmeldenummer: 01110924.6
(22) Anmeldetag: 05.05.2001
(51) Int. Cl.: A61K 9/107, A61K 9/48

(54) **Pharmazeutische Formulierungen enthaltend entzündungshemmende Wirkstoffe und deren Verwendung**
Pharmaceutical formulations comprising anti-inflammatory compounds and use thereof
Formulations pharmaceutiques comprenant des produits anti-inflammatoires et leur utilisation

(30) Priorität: 12.04.2001 EP 01109132
(43) Veröffentlichungstag der Anmeldung: 16.10.2002
(73) Patentinhaber: VESIFACT AG, 6342 Baar 2 (CH)
(72) Erfinder: Supersaxo, Andreas Werner, 6340 Baar (CH); Weder, Marc Antoine, 8803 Rüschlikon (CH); Weder, Hans Georg, 8803 Rüschlikon (CH)
(74) Vertreter: Hepp, Dieter

(56) Entgegenhaltungen:
- WO-A-01/28518
- WO-A-99/49848
- WO-A-99/56727

## Beschreibung

Die vorliegende Erfindung bezieht sich auf neue Formulierungen in Form von Mikroemulsion-Prekonzentraten und Mikroemulsionen, welche einen in Wasser schwerlöslichen Wirkstoff aus der Klasse der NSAIDs (non-steroidal antiinflammatory drugs) enthalten, sowie deren Anwendung.

Entzündungen entstehen unter anderem durch mechanische, thermische und chemische Verletzungen, Strahleneinwirkungen sowie Krankheitserreger (Bakterien und Viren), allergische beziehungsweise Autoimmunreaktionen. Ablauf und Ausbreitung der Entzündung gehen einher mit Reaktionen der körpereigenen Abwehr wie Antigen-Antikörper-Aktivierung sowie der Freisetzung von sogenannten Entzündungsmediatoren wie z. B. Kininen, Prostaglandinen, Leukotrienen, Histamin und Lymphokinen. Hier ist der Angriffspunkt von entzündungshemmenden Pharmaka, die sogenannten Antiphlogistika, die durch Eingriff in den Stoffwechsel der Mediatoren wirksam werden. Die Wirkung der Antiphlogistika, die in erster Linie als Antiarthritika und Antirheumatika eingesetzt werden, beruht zum Beispiel auf einer Hemmung der Prostaglandin-Biosynthese. Unter den nichtsteroidalen Antiphlogistika (NSAIDs) sind vor allem Pyrazol-Derivate, Oxicame sowie Arylessig- und -propionsäuren zu nennen. Zur letzteren Gruppe gehören unter anderen Indometacin, Diclofenac, Naproxen und Ibuprofen.

Ibuprofen wird seit mehr als 30 Jahren in der Rheuma- und Schmerztherapie eingesetzt. Neben der starken analgetischen und antiphlogistischen Wirksamkeit von Ibuprofen ist - im Vergleich zu anderen NSAIDs - besonders die gastrointestinale Verträglichkeit positiv herauszustellen.

Ibuprofen liegt als Wirkstoff mit einem Chiralitätszentrum sowohl in einer rechtsdrehenden R-(-) als auch linksdrehenden S-(+)-Form vor. Die R-Form unterliegt im menschlichen Körper einer intensiven enantiomeren Inversion zu dem analgetisch aktiven S-(+)-Ibuprofen oder Dexibuprofen.

In klassischen Darreichungsformen als Dragee oder Tablette wird der Wirkstoff Ibuprofen, der, wie oben bemerkt, zu den Arylpropionsäuren gehört, als freie Säure eingesetzt. Das schwach saure Ibuprofen mit dem pKₐ 4,4 ist in Wasser und im sauren Milieu des Magensaftes schlecht löslich. Erst in einem leicht alkalischen Milieu, wie z.B. im Dünndarm, gelingt die vollständige Auflösung. Letztere ist jedoch die Voraussetzung für eine schnelle Resorption und damit auch für einen schnellen Wirkungseintritt. Aus diesem Grund zeigen klassische Ibuprofensäure-haltige Dragees und Tabletten einen relativ verzögerten Wirkeintritt. Maximale Wirkstoffkonzentrationen werden im Blut erst 1-2 Stunden nach oraler Zufuhr erreicht.

Durch Einsatz von gepufferten Brausegranulaten oder -tabletten, die bereits vor der Einnahme aufgelöst werden und somit einen schnellen Transport vom Magen zum Resorptionsort Dünndarm gewährleisten, kann die Resorption beschleunigt werden. Den "brausenden" galenischen Formen gemeinsam ist jedoch eine gewisse Umständlichkeit der Zubereitung, da das Arzneimittel immer mit Wasser - welches nicht in jeder Situation zur Verfügung steht - aufgelöst werden muss, und/oder ein negatives Geschmackserlebnis. Aufgabe der vorliegenden Erfindung ist es, eine Formulierung mit analgetischer und entzündungshemmender Wirkung zu entwickeln, welche den notwendigen raschen Wirkungseintritt der Wirkstoff-Formulierung mit einer einfachen, angenehmen und geschmacksneutralen Einnahme verbindet. Überraschenderweise wurde festgestellt, dass Formulierungen auf der Basis eines Mikroemulsion-Prekonzentrates respektive einer Mikroemulsion die Resorptionsgeschwindigkeit von Ibuprofen nach oraler Gabe im Vergleich zu Dragees oder Tabletten signifikant erhöhten.

Unter dem erfindungsgemässen Mikroemulsion-Prekonzentrat wird ein System verstanden, das beim Kontakt mit Wasser und wässrigen Medien wie z.B. Magen-Darmsaft, z.B. bei Zugabe zu Wasser, eine Mikroemulsion ergibt. Bei einer solchen Mikroemulsion handelt es sich im herkömmlich anerkannten Sinn um eine nicht-opaque oder praktisch nicht-opaque kolloidale Dispersion, welche Wasser und organische Komponenten unter Einschluss lipophiler (d.h. hydrophober) Komponenten enthält.

Mikroemulsionen im Sinne der Erfindung lassen sich dadurch erkennen, dass sie eine oder mehrere der folgenden Eigenschaften aufweisen:
- Sie werden spontan gebildet, wenn ihre Komponenten miteinander in Kontakt gebracht werden; es ist also hierzu praktisch keine Zufuhr von Energie notwendig, und die Bildung solcher Mikroemulsionen erfolgt daher ohne Erhitzen oder Anwendung einer hohen Scherkraft oder einer anderen wesentlichen Durchmischung.
- Sie sind praktisch nicht opaque, nämlich transparent oder opaleszent, wenn sie unter einem optischen Mikroskop betrachtet werden. Sie sind in ihrem nichtgestörten Zustand optisch isotrop, obwohl sich bei einer Beobachtung beispielsweise unter Anwendung einer Röntgenstrahltechnik eine anisotrope Struktur feststellen lässt.
- Sie enthalten eine disperse oder stückige (Tröpfchen-) Phase, deren Teilchen eine Grösse von weniger als 200 nm haben, wovon ihre optische Transparenz herrührt. Die Teilchen können kugelig sein oder auch sonstige Strukturen haben; beispielsweise können sie flüssige Kristalle mit lamellaren, hexagonalen oder isotropen Symmetrien sein. Im allgemeinen enthalten Mikroemulsionen Tröpfchen oder Teilchen mit einer maximalen Abmessung, beispielsweise einem Durchmesser, von weniger als 150 nm, gewöhnlich etwa 10-100 nm.

Bei den eingangs erwähnten erfindungsgemässen Mikroemulsion-Prekonzentraten handelt es sich um galenische Systeme, die einen in Wasser schwerlöslichen Wirkstoff aus der Klasse der NSAIDs enthalten und beim Zusammenbringen mit Wasser bzw. Magen-Darmsaft spontan oder praktisch spontan, d.h. ohne nennenswerten Energieeintrag, zur Bildung einer Mikroemulsion befähigt sind.

Die erfindungsgemässen Mikroemulsion-Prekonzentrate sind in Auspruch 1 definiert. Sie sind vor allem dadurch gekennzeichnet, dass sie
(a) eine Mischung bestehend aus einem mittelkettigen Triglycerid und einer Omega-9-Fettsäure und/oder einer Omega-6-Fettsäure und
(b) eine oberflächenaktive Komponente enthaltend ein Tensid vom Polyoxyethylen-Typ und
(c) einen in Wasser schwerlöslichen, in Komponente (a) und/oder (b) jedoch löslichen therapeutischen Wirkstoff aus der Klasse der NSAIDs umfassen.

Das Verhältnis der Bestandteile (a): (b) : (c), (a) : (c) beziehungsweise (b) : (c) der erfindungsgemässen Mikroemulsion-Prekonznetrate muss selbstverständlich so gewählt werden, dass der Wirkstoff (c) stabil solubilisiert ist, d.h. es dürfen über mehrere Wochen keine Präzipitate auftreten.

Im Gegensatz zu den Formulierungen des Stands der Technik sind die Mikroemulsion-Prekonzentrate der vorliegenden Erfindung, trotz hohen Wirkstoffkonzentrationen, im wesentlichen frei von mit Wasser mischbaren oder in Wasser löslichen Komponenten. Dabei handelt es sich insbesondere um die Komponenten
- C₁-C₅-Alkyl- oder Tetrahydrofurfuryl-Diether oder -Teilether niedermolekularer Mono- oder Polyoxy-C₂-C₁₂-Alkandiole;
- 1,2-Propylenglykol;
- niedere Alkanole;
- Veresterungsprodukte von Polycarbonsäuren mit 2-10, insbesondere 3-5 Carboxylgruppen mit C₁-C₁₀-Alkoholen; und
- Veresterungsprodukte von Polyolen mit 2-10, insbesondere 3-5 Carboxylgruppen mit C₂-C₁₁-Carbonsäuren;
insbesondere im wesentlichen frei von Diethylenglykolmonomethylether, Glycofurol, 1,2-Propylenglykol, Triethylcitrat, Tributycitrat, Acetyltributylcitrat, Acetyltriethylcitrat, Triacetin, Ethanol, Polyethylenglykol, Dimethylisosorbit und Propylencarbonat.

Die erfindungsgemässen Mikroemulsion-Prekonzentrate können hergestellt werden, indem man die einzelnen Bestandteile, gegebenenfalls unter Erwärmen, innig miteinander vermischt. Die Mikroemulsion-Prekonzentrate können auch hergestellt werden, indem man die Komponente (b) unter Rühren, gegebenenfalls unter Erwärmen, in der Komponente (a) löst, und die erhaltene Lösung unter weiterem Rühren mit der Komponente (c) versetzt. Hierbei ist es von besonderer Bedeutung, dass die Komponente beziehungsweise der Wirkstoff (c) entweder in Komponente (a) oder Komponente (b) oder aber in beiden Komponenten (a) und (b) lösbar ist und dass beim Herstellen des Pre-Konzentrates, d.h. dem Gemisch aus allen drei Komponenten (a), (b) und (c) der Wirkstoff in jedem Fall weiterhin in gelöster Form vorliegt.

Als Komponente (a) eignen sich Mischungen aus einem mittelkettigen Fettsäuretriglycerid, zweckmässigerweise einem Fettsäuretriglycerid, in welchem die Fettsäurereste 4 bis 18, vorzugsweise 6 bis 18 C-Atome aufweisen, und einer Omega-9- und/oder einer Omega-6-Fettsäure. Diese Substanzen sind mit Wasser nicht mischbar oder in Wasser unlöslich bzw. praktisch unlöslich und weisen keine oder praktisch keine oberflächenaktive Funktion auf.

Bevorzugte mittelkettige Fettsäuretriglyceride sind Capryl-/Caprinsäure-Triglyceride, wie sie beispielsweise unter der Warenbezeichnung MIGLYOL bekannt und im Handel erhältlich sind (Fiedler, Lexikon der Hilfsstoffe, 3. Auflage, Seiten 808 bis 809, 1989). Hierzu gehören beispielsweise die folgenden Produkte:

### MIGLYOL 810, 812 und 818

Hierbei handelt es sich um ein fraktioniertes Kokosnussöl, das Triglyceride von Capryl- und Caprinsäure enthält und ein Molekulargewicht von etwa 520 (MIGLYOL 810 und 812) beziehungsweise 510 (MIGLYOL 818) hat. Es weist eine Fettsäure-Zusammensetzung mit C₆ von maximal 2 Prozent (MIGLYOL 810) und 3 Prozent (MIGLYOL 812 und 818) auf, mit C₈ etwa von 65 bis 75 Prozent (MIGLYOL 810), 50 bis 65 Prozent (MIGLYOL 812) und 45 bis 60 Prozent (MIGLYOL 818). C₁₀ ist bei MIGLYOL mit 25 bis 35 Prozent, bei MIGLYOL 812 mit etwa 30 bis 45 Prozent und bei MIGLYOL 818 etwa 25 bis 40 Prozent vertreten, C₁₂ mit maximal 2 Prozent (MIGLYOL 810), 5 Prozent (MIGLYOL 812) und 2 bis 5 Prozent (MIGLYOL 818). MIGLYOL 818 weist zusätzlich noch einen Anteil an C_{18:2} von etwa 4 bis 6 Prozent auf.

Ferner sind auch Triglyceride von Caprylsäure und Caprinsäure geeignet, wie sie unter der Warenbezeichnung MYRITOL bekannt und erhältlich sind (Fiedler, Lexikon der Hilfsstoffe, 3. Auflage, Seite 834, 1989). Hierzu gehört beispielsweise das Produkt MYRITOL 813.

Weitere geeignete Produkte dieser Klasse sind CAPTEX 355, CAPTEX 300, CAPTEX 800, CAPMUL MCT, NEOBEE M5 und MAZOL 1400. Geeignete Omega-9-Fettsäuren sind hauptsächlich solche mit -12-24, insbesondere 16-24, vorzugsweise 18-22 C-Atomen, beispielsweise Oelsäure und Eicosatriensäure. Besonders bevorzugt ist die Oelsäure.

Geeignete Omega-6-Fettsäuren sind hauptsächlich solche mit 12-24, insbesondere 16-24, vorzugsweise 18-22 C-Atomen, beispielsweise Linolsäure, gamma-Linolensäure, dihommo-gamma-Linolensäure und Arachidonsäure. Besonders bevorzugt ist die Linolsäure.

In einer besonders bevorzugten Ausführungsform verwendet man als Komponente (a) eine Mischung bestehend aus einem Capryl/Caprinsäure-Triglycerid, Oelsäure und/oder Linolsäure.

Bei Komponente (b), der oberflächenaktiven Komponente enthaltend ein Tensid vom Polyoxyethylen-Typ, kann es sich um ein hydrophiles oberflächenaktives Mittel oder um ein lipophiles oberflächenaktives Mittel handeln, doch kommen auch Gemische solcher Mittel in Frage.

Beispiele für solche Tenside sind folgende:
- Reaktionsprodukte von natürlichen oder hydrierten Pflanzenölen und Ethylenglykol, nämlich polyoxyethylenglykolierte natürliche oder hydrierte Pflanzenöle, wie polyoxyethylenglykolierte natürliche oder hydrierte Rizinusöle. Besonders geeignet sind die verschiedenen Tenside, die unter der Bezeichnung CREMOPHOR bekannt und erhältlich sind (Fiedler, Lexikon der Hilfsstoffe, 3. Auflage, Seiten 326 bis 327, 1989), vor allem die Produkte mit den Bezeichnungen CREMOPHOR RH 40, CREMOPHOR RH 60 und CREMOPHOR EL. Ferner eignen sich als solche Produkte auch die verschiedenen Tenside, die unter der Bezeichnung NIKKOL bekannt und erhältlich sind, beispielsweise NIKKOL HCO-60.
- Polyoxyethylensorbitanfettsäureester, beispielsweise die Mono- und Trilaurylester, die Mono- und Tripalmitylester, die Mono- und Tristearylester und die Mono- und Trioleylester, wie sie unter der Bezeichnung TWEEN bekannt und erhältlich sind (Fiedler, Lexikon der Hilfsstoffe, 3. Auflage, Seiten 1300 bis 1304, 1989), beispielsweise die Produkte
   TWEEN 20: Polyoxyethylen(20)sorbitanmonolaurat,
   TWEEN 40: Polyoxyethylen(20)sorbitanmonopalmitat,
   TWEEN 60: Polyoxyethylen(20)sorbitanmonostearat,
   TWEEN 80: Polyoxyethylen(20)sorbitanmonooleat,
   TWEEN 65: Polyoxyethylen(20)sorbitantristearat,
   TWEEN 85: Polyoxyethylen(20)sorbitantrioleat,
   TWEEN 21: Polyoxyethylen(4)sorbitanmonolaurat,
   TWEEN 61: Polyoxyethylen(4)sorbitanmonostearat und
   TWEEN 81: Polyoxyethylen(4)sorbitanmonooleat.

Besonders bevorzugt aus dieser Klasse von Verbindungen ist TWEEN 80.
- Polyoxyethylenfettsäureester, beispielsweise die im Handel unter der Bezeichnung MYRJ bekannten und erhältlichen Polyoxyethylenstearinsäureester (Fiedler, Lexikon der Hilfsstoffe, 3. Auflage, Seite 834, 1989), insbesondere das Produkt MYRJ 52, sowie auch die unter der Bezeichnung CETIOL HE bekannten und erhältlichen Polyoxyethylenfettsäureester (Fiedler, Lexikon der Hilfsstoffe, 3. Auflage, Seite 284, 1989).
- Copolymerisate von Polyoxyethylen und Polyoxypropylen, wie sie beispielsweise unter den Bezeichnungen PLURONIC und EM-KALYX bekannt und erhältlich sind (Fiedler, Lexikon der Hilfsstoffe, 3. Auflage, Seiten 956 bis 958, 1989), insbesondere das Produkt PLURONIC F68.
- Blockcopolymerisate von Polyoxyethylen und Polyoxypropylen, wie sie beispielsweise unter der Bezeichnung POLOXAMER bekannt und erhältlich sind (Fiedler, Lexikon der Hilfsstoffe, 3. Auflage, Seite 959, 1989), insbesondere das Produkt POLOXAMER 188.
- Polyethoxylierte Vitamin E Derivate, insbesondere das Produkt VITAMIN E TPGS (d-Alpha Tocoperyl Polyethylene Glycol 1000 Succinate, Eastman).
- Polyethoxylierte Hydroxyfettsäureester, insbesondere das Produkt SOLUTOL HS 15 (Polyoxyethylen-660-Hydroxystearat, BASF).
- Umesterungsprodukte von natürlichen Pflanzenölglyceriden und Polyethylenpolyolen. Hierzu gehören Umesterungsprodukte aus verschiedenen, beispielsweise nichthydrierten, Pflanzenölen, wie Maisöl, Kernöl, Mandelöl, Erdnussöl, Olivenöl und Palmenöl, sowie aus Gemischen hiervon mit Polyethylenglykolen, insbesondere mit solchen, die ein mittleres Molekulargewicht von 200-800 haben. Verschiedene derartige Umesterungsprodukte sind unter der Bezeichnung LABRAFIL bekannt und erhältlich (Fiedler, Lexikon der Hilfsstoffe, 3. Auflage, Seite 707, 1989); hiervon sind die Produkte LABRAFIL M 1944 CS und LABRAFIL M 2130 CS besonders geeignet.
- Ethylenoxidaddukte von Sterolen und Derivaten davon, beispielsweise von Cholesterol und Derivaten hiervon, wie Produkte, die von Sitosterol, Campesterol, oder Stigmasterol abgeleitet sind, beispielsweise von Sojasterolen und Derivaten hiervon (Fiedler, Lexikon der Hilfsstoffe, 3. Auflage, Seiten 554 und 555, 1989), wie sie unter den Bezeichnungen GENEROL bekannt und erhältlich sind, insbesondere die Produkte GENEROL 122 E5, 122 E10 und 122 E25.

Die erfindungsgemässen Mikroemulsion-Prekonzentrate umfassen sowohl Systeme, die ein einzelnes oberflächenaktives Mittel enthalten, als auch Systeme, die ein Gemisch von zwei oder mehreren oberflächenaktiven Mitteln enthalten, z.B. TWEEN 80 + CREMOPHOR RH 40, TWEEN 80 + CREMOPHOR RH 40 + VITAMIN E TPGS etc.

Erfindungsgemäss verwendet man vorzugsweise eine oberflächenaktive Komponente, welche einen Polyoxyethylensorbitanfettsäureester, ein polyoxyethylen-glykoliertes natürliches oder hydriertes Pflanzenöl oder Mischungen davon enthält.

Komponente (c), der in Wasser schwerlösliche, in Komponente (a) und/oder (b) jedoch lösliche therapeutische Wirkstoff aus der Klasse der NSAIDs, ist vorzugsweise Ibuprofen, Dexibuprofen oder Naproxen; es kann aber auch ein anderes geeignetes NSAIDs, gegebenenfalls in Kombination mit Antioxidantien wie z.B. Vitamin E verwendet werden. Beispiele von anderen geeigneten NSAIDs sind: Anthranilsäure-Derivate, Essigsäure-Derivate, Oxicame, Propionsäure-Derviate, Pyrazolon-Derivate, Salicylsäure-Derivate und selektive COX-2-Hemmer (vgl. Arzneimittel Kompendium der Schweiz 2001, Documed AG, CH-4010 Basel, Herausgeber: Jürg Morant und Hans Ruppanner).

Die erfindungsgemässen Mikroemulsion-Prekonzentrate können auch noch weitere Stoffe enthalten, wie z.B. Antioxidantien, Verdikkungsmittel, Geruchs- und/oder Geschmacksstoffe, Farbstoffe, etc.

Die erfindungsgemässen Mikroemulsion-Prekonzentrate sind in erster Linie für eine orale Anwendung gedacht. Bevorzugt wird dabei die sogenannte Einheitsdosisform, d.h. das Mikroemulsion-Prekonzentrat wird in einen Formkörper wie eine Weich- oder Hartkapsel z.B. aus Gelatine oder Stärke, verbracht. Wenn die den Wirkstoff enthaltende Pre-Mikroemulsion freigesetzt wird, bildet sich spontan in Verbindung mit Magen-Darmflüssigkeit eine Mikroemulsion. Die erfindungsgemässen Zusammensetzungen erweisen sich für eine orale Verabreichung in Form von Einheitsdosisformem auch deshalb als besonders geeignet, weil eine Zugabe von flüchtigen organischen Lösungsmitteln, insbesondere vom häufig verwendeten Ethanol nicht erforderlich ist. Beim Einsatz der genannten Lösungsmittel wird durch deren Verdampfen durch die Aussenwand des Formkörpers, insbesondere der Weich- oder Hartgelatinekapsel, die Lagerfähigkeit negativ beeinträchtigt und der Wirkstoff kristallisiert aus. Das Eintreten dieser negativen Effekte muss durch aufwendige Massnahmen bei der Verpackung und Lagerung vermieden werden.

Die neuen Zusammensetzungen lassen sich auch zu Brausetabletten oder zu Granulate weiterverarbeiten.

Die erfindungsgemässen Mikroemulsion-Prekonzentrate enthalten zweckmässigerweise 5 bis 45 vorzugsweise 20 bis 40 Gewichtsprozent eines in Wasser schwerlöslichen, in Komponente (a) und/oder (b) jedoch löslichen therapeutischen Wirkstoffs aus der Klasse der NSAIDs (Komponente (c)), 5 bis 60 vorzugsweise 15 bis 40 Gewichtsprozent einer Mischung bestehend aus einem mittelkettigen Triglycerid und einer Omega-9-Fettsäure und/oder einer Omega-6-Fettsäure (Komponente (a)) und 20 bis 90 vorzugsweise 25 bis 65 Gewichtsprozent der oberflächenaktiven Komponente (b).

Durch die vorliegende Erfindung lassen sich auch pharmazeutische Zusammensetzungen bereitstellen, die einen in Wasser schwerlöslichen, in Komponente (a)und/oder (b) jedoch löslichen therapeutischen Wirkstoff aus der Klasse der NASIDs enthalten und die selbst Mikroemulsionen darstellen; in diesen Mikroemulsionen ist der Wirkstoff stabil solubilisiert, wobei über mehrere Wochen keine Präzipitate beobachtet werden. Für eine orale Verabreichung können Mikroemulsionen, die man beispielsweise durch Verdünnen der erfindungsgemässen Mikroemulsion-Prekonzentrate mit Wasser oder einem wässrigen Medium erhält, direkt als Trinkformulierungen verwendet werden. Ist eine topische oder parenterale Verabreichung vorgesehen, dann enthalten Zusammensetzungen, in denen weitere Hilfsstoffe vorhanden sein können, ebenfalls Wasser, so dass sich eine wässrige Mikroemulsion in Form eines Sprays, eines Gels, einer Lotion, einer Creme, eines Plasters, eines Roll-on einer Injektionslösung, einer Infusionslösung oder dergleichen ergibt. Solche pharmazeutischen Zusammensetzungen in Form von Mikroemulsionen sind ebenfalls neu und Gegenstand der vorliegenden Erfindung.

Die erfindungsgemässen Mikroemulsionen sind vor allem dadurch gekennzeichnet, dass sie erhältlich sind durch Vermischen eines Mikroemulsion-Prekonzentrats der vorstehend beschriebenen Zusammensetzungen mit Wasser oder einem wässrigen Medium. Beim Zusammenbringen des Pre-Konzentrats mit Wasser bzw. Magen- und Darmsaft wird spontan oder praktisch spontan, d.h. ohne nennenswerten Energieeintrag eine Mikroemulsion gebildet.

Je nach der Menge des vorhandenen Wassers handelt es sich um W/O-Mikroemulsionen, um bikontinuierliche Mikroemulsionen oder O/W-Mikroemulsionen.

Die erfindungsgemässen Mikroemulsionen vom O/W-Typ (Öl-in-Wasser) weisen Stabilitätseigenschaften auf, wie sie weiter oben im Zusammenhang mit Mikroemulsionen beschrieben worden sind, d.h. insbesondere, dass in diesen Mikroemulsionen der Wirkstoff stabil solubilisiert ist und über mehrere Wochen kein Präzipitat beobachtet werden kann. Die Teilchengrösse dieser Mikroemulsionen ist kleiner als 150 nm, vorzugsweise kleiner als 100 nm.

Durch die folgenden Beispiele werden die erfindungsgemässen Zusammensetzungen weiter erläutert. Die Beispiele 1.1 bis 1.5 zeigen die Herstellung von Zusammensetzungen, die sich beispielsweise zur oralen Schmerz- und Rheumatherapie eignen. Beispiele 2.1 bis 2.4 zeigen die Herstellung von Zusammensetzungen, die sich beispielsweise zur topischen Behandlung von Rheuma eignen. Beispiel 3.1 zeigt die Herstellung einer Zusammensetzung für eine parenterale Anwendung, die sich beispielsweise zur subkutanen oder intramuskulären Behandlung von entzündlichen Schmerzen eignet. In Beispiel 4 werden die pharmakokinetischen Parameter (cₘₐₓ, tₘₐₓ, AUC) einer erfindungsgemässen Formulierung, welche oral in Weichgelatinekapseln verabreicht wurde, ermittelt und mit denjenigen von Dragees verglichen.

Die Beispiele werden unter besonderer Bezugnahme auf Ibuprofen und Dexibuprofen beschrieben. Durch Anwendung anderer geeigneter NSAIDs lassen sich jedoch vergleichbare Zusammensetzungen herstellen.

### Beispiel 1: Herstellung oraler Ibuprofen oder Dexibuprofen Dosierungsformen vom Typ Mikroemulsion-Prekonzentrat

### Beispiel 1.1

| | |
|---|---|
| Ibuprofen (c1) | 20.00% |
| Miglyol 812 (a1) | 20.00% |
| Oelsäure (a2) | 5.00% |
| Tween 80 (b1) | 37.50% |
| Cremophor RH 40 (b2) | 12.50% |
| Vitamin E Acetat(c2) | 5.00% |

Das Ibuprofen wird unter Rühren bei Raumtemperatur, gegebenenfalls unter leichtem Erwärmen, in den Komponenten (a1), (a2), (b1), (b2) und (c2) gelöst. Das gebildete Mikroemulsion-Prekonzentrat wird in eine Weich- oder Hartkapsel aus Gelatine oder Stärke abgefüllt.

Alternativ kann das Mikroemulsion-Prekonzentrat auch in einen Dispenser abgefüllt werden. In diesem Fall stellt der Patient durch entsprechende Verdünnung mittels Wasser oder einer anderen wässrigen Flüssigkeit aus dem Mikroemulsion-Prekonzentrat eine orale Trinklösung vom Typ O/W-Mikroemulsion her.

In analoger Weise lassen sich auch folgende Zusammensetzungen herstellen und in Kapseln oder in Dispenser abfüllen.

### Beispiel 1.2

| | |
|---|---|
| Ibuprofen (c) | 20.00% |
| Miglyol 812 (a1) | 25.00% |
| Oelsäure (a2) | 5.00% |
| Tween 80 (b1) | 37.50% |
| Cremophor RH 40 (b2) | 12.50% |

### Beispiel 1.3

| | |
|---|---|
| Ibuprofen (c1) | 20.00% |
| Miglyol 812 (a1) | 20.00% |
| Oelsäure (a2) | 5.00% |
| Tween 80 (b1) | 37.50% |
| Cremophor EL (b2) | 12.50% |
| Vitamin E Acetat (c2) | 5.00% |

### Beispiel 1.4

| | |
|---|---|
| Ibuprofen (c) | 10.00% |
| Miglyol 812 (a1) | 35.00% |
| Oelsäure (a2) | 5.00% |
| Tween 80 (b1) | 37.50% |
| Cremophor EL (b2) | 12.50% |

### Beispiel 1.5

| | |
|---|---|
| Dexibuprofen (c1) | 30.00% |
| Miglyol 812 (a1) | 17.50% |
| Oelsäure (a2) | 4.40% |
| Tween 80 (b1) | 32.80% |
| Cremophor RH 40 (b2) | 10.90% |
| Vitamin E Acetat (c2) | 4.40% |

Werden Zusammensetzungen obiger Art mit künstlichem Magen- oder Darmsaft z.B. auf 1:100 verdünnt, entstehen Mikroemulsionen, die im Falle repräsentativer Beispiele die folgenden Teilchengrössen aufweisen (vgl. Tabelle 1):

**Tabelle 1**

| Zusammensetzung Mikroemulsion-Prekonzentrat | Partikeldurchmesser¹⁾ der O/W-Mikroemulsion | |
|---|---|---|
| | Magensaft [nm] | Darmsaft [nm] |
| Beispiel 1.1 | 17.9 ± 7.2 | 18.6 ± 6.0 |
| Beispiel 1.2 | 25.6 ± 10.1 | 18.8 ± 6.5 |
| Beispiel 1.3 | 20.4 ± 8.2 | 19.7 ± 4.7 |
| Beispiel 1.4 | 25.0 ± 6.7²⁾ | - |
| Beispiel 1.5 | 56.3 ± 33.2 | 18.3 ± 6.6 |

| | | |
|---|---|---|
| 1) Die Partikeldurchmesser und Partikelgrössenverteilungen wurden mittels dynamischer Laserlicht-Streuungsmessungen bestimmt (Gerät: Nicomp 370 Submicron Particle Sizer, Auswertung: Volume weighting). | | |
| 2) Diese Ibuprofen Mikroemulsionen wurden durch eine 1:10 Verdünnung des Mikroemulsion-Prekonzentrates mit 10 mM Phosphatpuffer pH 6 bei Raumtemperatur gebildet. | | |

Aus der nachfolgenden Tabelle ist ersichtlich, dass die Mikroemulsionsbildung der Mikroemulsion-Prekonzentrate nach Abfüllung sowie Lagerung in Weichgelatinekapseln (WGK) unverändert bleibt.

**Tabelle 2**

| Mikroemulsion-Prekonzentrat Beispiel 1.1 Charge 201111 | Partikeldurchmesser der Ibuprofen Mikroemulsion¹⁾ | |
|---|---|---|
| | Magensaft [nm] | Darmsaft [nm] |
| Vor Abfüllung in WGK | 17.9 ± 7.2 | 18.6 ± 6.0 |
| Nach Abfüllung in WGK | 19.8 ± 7.0 | 20.6 ± 5.3 |
| Nach 1-monatiger Lagerung in WGK bei 25°C und 60%RH | 16.9 ± 6.3 | 20.0 ± 5.4 |
| Nach 1-monatiger Lagerung in WGK bei 40°C und 75%RH | 17.7 ± 6.8 | 19.8 ± 5.5 |
| Nach 3-monatiger Lagerung in WGK bei 25°C und 60%RH | 13.6 ± 6.9 | 18.4 ± 5.9 |

| | | |
|---|---|---|
| 1) Die Ibuprofen Mikroemulsionen wurden durch eine 1:100 Ver dünnung der Mikroemulsion-Prekonzentrate mit künstlichem Ma gen- und Darmsaft bei 37°C gebildet. In WGK abgefüllte Mi kroemulsion-Prekonzentrate wurden zur Mikroemulsionsbildung der WGK entnommen. Die Partikeldurchmesser und Partikelgrö ssenverteilungen der erhaltenen Ibuprofen Mikroemulsionen wurden mittels dynamischer Laserlicht-Streuungsmessungen bestimmt (Gerät: Nicomp 370 Submicron Particle Sizer, Auswer tung: Volume weighting). | | |

### Beispiel 2: Herstellung topisch anwendbarer Ibuprofen Formen vom Typ Mikroemulsion

Die in Beispiel 1.1 bis 1.5 beschriebenen Mikroemulsion-Prekonzentrate dienen nachfolgend als Basis zur Herstellung von Sprays, Gelen, Cremen und anderen topischen Darreichungsformen, indem sie mit weiteren Additiven wie Wasser, Verdickungsmitteln und dergleichen kombiniert werden.

### Beispiel 2.1: Ibuprofen 1.0% Mikroemulsions Pumpspray

| | |
|---|---|
| Mikroemulsion-Prekonzentrat gemäss Beisp. 1.4 | 10.00% |
| Na₂-EDTA | 0.05% |
| Benzalkoniumchlorid | 0.10% |
| 10 mM Phosphatpuffer pH 6 ad | 100.00% |

Das Mikroemulsion-Prekonzentrat wird unter Rühren bei Raumtemperatur dem Phosphatpuffer, enthaltend Na₂-EDTA und Benzalkoniumchlorid, zugesetzt. Die resultierende Ibuprofen O/W-Mikroemulsion wird in einen Pumpspray abgefüllt. Anstelle des Pumpspray kommen auch Druckgas- oder Aerosolsprays in Frage.

### Beispiel 2.2: Ibuprofen 1.0% Hydrogel

| | |
|---|---|
| Mikroemulsion-Prekonzentrat gemäss Beisp. 1.4 | 10.00% |
| Na₂-EDTA | 0.05% |
| Benzalkoniumchlorid | 0.10% |
| Natriumcarboxymethylcellulose 450 cP | 3.50% |
| 10 mM Phosphatpuffer pH 6 ad | 100.00% |

Das Mikroemulsion-Prekonzentrat wird unter Rühren zum Phosphatpuffer, enthaltend Na₂-EDTA und Benzalkoniumchlorid, zugesetzt. Die resultierende Ibuprofen O/W-Mikroemulsion wird in üblicher Weise mit der Natrium-Carboxymethylcellulose zum Hydrogel weiter verarbeitet und konfektioniert.

### Beispiel 2.3: Ibuprofen 1.0% O/W-Emulsion

| | |
|---|---|
| Mikroemulsion-Prekonzentrat gemäss Beisp. 1.4 | 10.000% |
| Isopropylpalmitat | 8.000% |
| Glycerylstearat | 7.000% |
| Glycerin | 5.000% |
| Steareth-2 + PEG-8 Distearate | 4.000% |
| Paraffinum liquidum | 4.000% |
| Cera microcristallina | 4.000% |
| Steareth-21 | 3.000% |
| Dimethicon | 1.000% |
| Suttocide A | 0.250% |
| Lanolinalkohol | 0.100% |
| Natriumhydroxid | 0.005% |
| Wasser ad | 100.000% |

Das Mikroemulsion-Prekonzentrat wird unter Rühren bei Raumtemperatur zur Wasserphase gegeben. Die resultierende Ibuprofen O/W-Mikroemulsion wird in üblicher Weise mit der Oelphase zur O/W-Emulsion weiter verarbeitet und konfektioniert.

### Beispiel 2.4: Ibuprofen 1.0% Mikroemulsions Roll-on

| | |
|---|---|
| Mikroemulsion-Prekonzentrat gemäss Beisp. 1.4 | 10.00% |
| Na₂-EDTA | 0.05% |
| Benzalkoniumchlorid | 0.10% |
| 10 mM Phosphatpuffer pH 6 ad | 100.00% |

Das Mikroemulsion-Prekonzentrat wird unter Rühren bei Raumtemperatur dem Phosphatpuffer, enthaltend Na₂-EDTA und Benzalkoniumchlorid, zugesetzt. Die resultierende Ibuprofen O/W-Mikroemulsion wird in einen Roll-on abgefüllt.

### Beispiel 3: Herstellung parenteral anwendbarer Ibuprofen Formen vom Typ Mikroemulsion

Die in Beispiel 1.1 bis 1.5 beschriebenen Mikroemulsion-Prekonzentrate können als Basis zur Herstellung von Injektionslösungen dienen, indem sie mit weiteren Additiven, wie physiologischer Kochsalzlösung oder 5%iger Glukoselösung und dergleichen, entsprechend verdünnt werden.

### Beispiel 3.1: Ibuprofen 0,1% Injektionslösung

| | |
|---|---|
| Mikroemulsion-Prekonzentrat gemäss Beispiel 1.4 | 1.00% |
| 5%ige Glukoselösung ad | 100.00% |

Das Mikroemulsion-Prekonzentrat wird unter Rühren bei Raumtemperatur der Glukoselösung zugesetzt. Die resultierende Ibuprofen O/W-Mikroemulsion wird 0.2 µm steril filtriert und in gebräuchliche sterile Gebinde abgefüllt.

### Beispiel 4: Pharmakokinetik des Ibuprofen Mikroemulsion-Prekonzentrates gemäss Beispiel 1.1 nach oraler Verabreichung in Weichkapseln aus Gelatine und Stärke

Ziel dieser Studie war es, die Pharmakokinetik einer oralen Einzeldosis von 2 x 200 mg Ibuprofen, welche in Form des Mikroemulsion-Prekonzentrates gemäss Beispiel 1.1 in Weichkapseln aus Gelatine und Stärke appliziert wurde, zu ermitteln.

### Präparationen

| | |
|---|---|
| A | Weichgelatinekapseln enthaltend das Ibuprofen Mikroemulsion-Prekonzentrat gemäss Beispiel 1.1 Wirkstoffgehalt: 200 mg Ibuprofen pro Kapsel |
| | |
| B | Stärkekapseln enthaltend das Ibuprofen Mikroemulsion-Prekonzentrat gemäss Beispiel 1.1 Wirkstoffgehalt: 200 mg Ibuprofen pro Kapsel |

Dosierung: 2 x 200 mg Ibuprofen, oral in 2 Kapseln

Einnahme
Morgens, nüchtern

Probanden: n = 4

Messparameter: Plasmaspiegel von Ibuprofen [µg/ml Plasma]

### Resultate

Aus den Plasmaspiegelverläufen der erfindungsgemässen Testpräparate A und B ist ersichtlich, dass die maximalen Ibuprofen Plasmaspiegel von 45.3 und 49.0 µg/ml nach 0.68 und 0.63 Stunden erreicht werden (vgl. Figur 1). Damit weisen die Testpräparate A und B im Vergleich zu kommerziell erhältlichen Ibuprofen 200 mg

Dragees deutliche Unterschiede hinsichtlich der Anflutungsgeschwindigkeit und des erreichten Maximums auf (vgl. Tabelle 3).

**Tabelle 3**

| Parameter | Testpräparat A | Testpräparat B | Ibuprofen 200 mg Dragees¹⁾ |
|---|---|---|---|
| cₘₐₓ [µg/ml] | 45.3 | 49.0 | 32.0 |
| tₘₐₓ [h] | 0.68 | 0.63 | 1.30 |
| AUC_{inf} [µg/ml/h] | 123 | 121 | 108.0 |

| | | | |
|---|---|---|---|
| 1) Literaturdaten aus einer analog durchgeführten Studie | | | |

## Patentansprüche

1. Zusammensetzung in Form eines Mikroemulsion-Prekonzentrates enthaltend
(a) eine Mischung bestehend aus einem Triglycerid und einer Omega-9-Fettsäure und/oder einer Omega-6-Fettsäure; und
(b) eine oberflächenaktive Komponente enthaltend ein Tensid,
(c) einen Wirkstoff ausgewählt aus der Klasse der nicht steroidalen Entzündungshemmer, wobei der Wirkstoff in (a) und/oder (b) lösbar ist.

2. Zusammensetzung nach Anspruch 1, **dadurch gekennzeichnet, dass** als Triglycerid ein mittelkettiges Triglycerid verwendet wird.

3. Zusammensetzung nach einem der Ansprüche 1 und 2, **dadurch gekennzeichnet, dass** als Tensid ein Tensid vom Polyoxyethylen-Typ verwendet wird.

4. Zusammensetzung nach einem der Ansprüche 1 bis 3, wobei der Wirkstoff ausgewählt ist aus der Gruppe bestehend aus Heteroaryl- sowie Aryl-Essig und -Propionsäuren und der Gruppe der COX-2 Hemmer.

5. Zusammensetzung nach einem der Ansprüche 1 bis 3, wobei der Wirkstoff ausgewählt ist aus der Gruppe bestehen aus Indometacin, Diclofenac, Naproxen, Ibuprofen, Dexibuprofen sowie Celeoxib und Rofaecoxib.

6. Zusammensetzung nach einem der Ansprüche 1 bis 3, **dadurch gekennzeichnet, dass** der Wirkstoff Ibuprofen ist.

7. Zusammensetzung nach einem der Ansprüche 1 bis 3, **dadurch gekennzeichnet, dass** der Wirkstoff Dexibuprofen ist.

8. Zusammensetzung nach einem der Ansprüche 1 bis 3, **dadurch gekennzeichnet, dass** der Wirkstoff Naproxen ist.

9. Zusammensetzung in Form einer Mikroemulsion erhältlich durch Vermischen eines Mikroemulsions-Prekonzentrats gemäss einer der Ansprüche 1 bis 8 mit Wasser oder einem wässrigen Medium.

10. Zusammensetzung nach einem der Ansprüche 1 bis 9, enthaltend zusätzliche Komponenten die nicht den nachfolgenden Stoff-Gruppen angehören:
- C₁-C₅-Alkyl- oder Tetrahydrofurfuryl-Diether oder
- Teilether niedermolekularer Mono- oder Polyoxy-C₂-C₁₂-Alkandiole;
- 1,2-Propylenglykol;
- niedere Alkanole;
- Veresterungsprodukte von Polycarbonsäuren mit 2-10, insbesondere 3-5 Carboxylgruppen mit C₁-C₁₀-Alkoholen; und
- Veresterungsprodukte von Polyolen mit 2-10, insbesondere 3-5 Carboxylgruppen mit C₂-C₁₁-Carbonsäuren.

11. Zusammensetzung nach einem der Ansprüche 2 bis 10, **dadurch gekennzeichnet, dass** die Fettsäurereste des mittelkettigen Triglycerids 4-18 C-Atome aufweisen.

12. Zusammensetzung nach einem der Ansprüche 2 bis 10, **dadurch gekennzeichnet, dass** die Fettsäurereste des mittelkettigen Triglycerids 6-18 C-Atome aufweisen.

13. Zusammensetzung nach Anspruch 11 oder 12, **dadurch gekennzeichnet, dass** das mittelkettige Triglycerid ein Capryl/Caprinsäure-Triglycerid ist.

14. Zusammensetzung nach einem der Ansprüche 1 bis 13, **dadurch gekennzeichnet, dass** die Omega-9-Fettsäure und/oder die Omega-6-Fettsäure 12-24 C-Atome aufweist.

15. Zusammensetzung nach einem der Ansprüche 1 bis 13, **dadurch gekennzeichnet, dass** die Omega-9-Fettsäure und/oder die Omega-6-Fettsäure 16-24 C-Atome aufweist.

16. Zusammensetzung nach einem der Ansprüche 1 bis 13, **dadurch gekennzeichnet, dass** die Omega-9-Fettsäure und/oder die Omega-6-Fettsäure 18-22 C-Atome aufweist.

17. Zusammensetzung nach einem der Ansprüche 1 bis 16, **dadurch gekennzeichnet, dass** die Omega-9-Fettsäure Oelsäure ist.

18. Zusammensetzung nach einem der Ansprüche 1 bis 17, **dadurch gekennzeichnet, dass** die Omega-6-Fettsäure Linolsäure ist.

19. Zusammensetzung nach einem der Ansprüche 1 bis 18, **dadurch gekennzeichnet, dass** sie als Komponente (a) eine Mischung aus einem Capryl-/Caprinsäure-Triglycerid, Ölsäure und/oder Linolsäure enthält.

20. Zusammensetzung nach einem der Ansprüche 1 bis 19, **dadurch gekennzeichnet, dass** das Mengenverhältnis von Omega-9-Fettsäure und/oder Omega-6-Fettsäure zum mittelkettigen Triglycerid 1:1 bis 1:200 ist.

21. Zusammensetzung nach einem der Ansprüche 1 bis 19, **dadurch gekennzeichnet, dass** das Mengenverhältnis von Omega-9-Fettsäure und/oder Omega-6-Fettsäure zum mittelkettigen Triglycerid 1:2 bis 1:20 ist.

22. Zusammensetzung nach einem der Ansprüche 1 bis 20, **dadurch gekennzeichnet, dass** die oberflächenaktive Komponente (b) ein Polyoxyethylensorbitanfettsäureester, ein polyoxyethylen-glykoliertes natürliches oder hydriertes Pflanzenöl oder Mischungen davon enthält.

23. Zusammensetzung nach einem der Ansprüche 1 bis 8 und 10 bis 22, **dadurch gekennzeichnet, dass** die Komponente (a) in einer Menge von 5 bis 60 Gewichtsprozent bezogen auf das Gesamtgewicht der Zusammensetzung vorhanden ist.

24. Zusammensetzung nach einem der Ansprüche 1 bis 8 und 10 bis 23, **dadurch gekennzeichnet, dass** die oberflächenaktive Komponente (b) in einer Menge von 20 bis 90 Gewichtsprozent bezogen auf das Gesamtgewicht der Zusammensetzung vorhanden ist.

25. Zusammensetzung nach einem der Ansprüche 9 bis 24, **dadurch gekennzeichnet, dass** sie eine O/W-Mikroemulsion mit einer durchschnittlichen Teilchengrösse unter 150 nm ist.

26. Zusammensetzung nach einem der Ansprüche 9 bis 24, **dadurch gekennzeichnet, dass** sie eine O/W-Mikroemulsion mit einer durchschnittlichen Teilchengrösse unter 100 nm ist.

27. Formkörper zur oralen Verabreichung enthaltend einen Wirkstoff aus der Klasse der nicht steroidalen Entzündungshemmer in einer Zusammensetzung nach einem der Ansprüche 1 bis 26 zur Darreichung des Wirkstoffs.

28. Formkörper nach Anspruch 27, **dadurch gekennzeichnet, dass** er ein Biopolymer enthält.

29. Formkörper nach Anspruch 27, **dadurch gekennzeichnet, dass** das Biopolymer Gelatine ist.

30. Zusammensetzung enthaltend einen Wirkstoff aus der Klasse der nicht steroidalen Entzündungshemmer nach einem der Ansprüche 1 bis 26 zur topischen Verabreichung.

31. Zusammensetzung enthaltend einen Wirkstoff aus der Klasse der nicht steroidalen Entzündungshemmer nach einem der Ansprüche 1 bis 26 zur kutanen Verabreichung.

## Claims

1. Composition in the form of a micro-emulsion pre-concentrate containing
(a) a mixture consisting of a triglyceride and an omega-9 fatty acid and/or an omega-6 fatty acid; and
(b) a surface-active component containing a tenside;
(c) an active principle selected from the class of non-steroidal anti-inflammatory drugs, the active principle being soluble in (a) and/or (b).

2. Composition according to claim 1, **characterised in that** the triglyceride used is a medium-chained triglyceride.

3. Composition according to either of claims 1 and 2, **characterised in that** the tenside used is a tenside of the polyoxyethylene type.

4. Composition according to any of claims 1 to 3, **characterised in that** the active principle is selected from the group comprising heteroaryl and aryl acetic and propionic acids and the group comprising the COX-2 inhibitors.

5. Composition according to any of claims 1 to 3, **characterised in that** the active principle is selected from the group comprising indometacin, diclofenac, naproxen, ibuprofen, dexibuprofen and celecoxib and rofecoxib.

6. Composition according to any of claims 1 to 3, **characterised in that** the active principle is ibuprofen.

7. Composition according to any of claims 1 to 3, **characterised in that** the active principle is dexibuprofen.

8. Composition according to any of claims 1 to 3, **characterised in that** the active principle is naproxen.

9. Composition in the form of a micro-emulsion obtainable by blending a micro-emulsion pre-concentrate according to any of claims 1 to 8 with water or an aqueous medium.

10. Composition according to any of claims 1 to 9, containing additional components not belonging to the following groups of substances:
- C₁-C₅ alkyl or tetrahydrofurfuryl diethers or
- partial ethers of lower-molecular mono or polyoxy C₂-C₁₂ alkane dioles;
- 1,2-propylene glycol;
- lower alkanols;
- esterification products of polycarbonic acids having 2 to 10, especially 3 to 5 carboxyl groups with C₁ - C₁₀ alcohols; and
- esterification products of polyols having 2 to 10, especially 3 to 5 carboxyl groups with C₂ to C₁₁ carbonic acids.

11. Composition according to any of claims 2 to 10, **characterised in that** the fatty acid radicals of the medium-chain triglyceride have 4 to 18 carbon atoms.

12. Composition according to any of claims 2 to 10, **characterised in that** the fatty acid radicals of the medium-chain triglyceride have 6 to 18 carbon atoms.

13. Composition according to claim 11 or 12, **characterised in that** the medium-chain triglyceride is a caprylic/capric triglyceride.

14. Composition according to any of claims 1 to 13, **characterised in that** the omega-9 fatty acid and/or the omega-6 fatty acid has 12 to 24 carbon atoms.

15. Composition according to any of claims 1 to 13, **characterised in that** the omega-9 fatty acid and/or the omega-6 fatty acid has 16 to 24 carbon atoms.

16. Composition according to any of claims 1 to 13, **characterised in that** the omega-9 fatty acid and/or the omega-6 fatty acid has 18 to 22 carbon atoms.

17. Composition according to any of claims 1 to 16, **characterised in that** the omega-9 fatty acid is oleic acid.

18. Composition according to any of claims 1 to 17, **characterised in that** the omega-6 fatty acid is linoleic acid.

19. Composition according to any of claims 1 to 18, **characterised in that** as component (a) it contains a mixture of a caprylic/capric triglyceride, oleic acid and/or linoleic acid.

20. Composition according to any of claims 1 to 19, **characterised in that** the quantity ratio of omega-9 fatty acid and/or omega-6 fatty acid to the medium-chain triglyceride is 1:2 to 1:200.

21. Composition according to any of claims 1 to 19, **characterised in that** the quantity ratio of omega-9 fatty acid and/or omega-6 fatty acid to the medium-chain triglyceride is 1:1 to 1:20.

22. Composition according to any of claims 1 to 20, **characterised in that** surface-active component (b) contains a polyoxyethylene sorbitan fatty acid ester, a polyoxyethylene-glycolised natural or hydrated vegetable oil, or mixtures thereof.

23. Composition according to any of claims 1 to 8 and 10 to 22, **characterised in that** component (a) is present in a quantity of 5 to 60 percent by weight, based on the overall weight of the composition.

24. Composition according to any of claims 1 to 8 and 10 to 23, **characterised in that** surface-active component (b) is present in a quantity of 20 to 90 percent by weight, based on the overall weight of the composition.

25. Composition according to any of claims 9 to 24, **characterised in that** it is an O/W micro-emulsion having an average particle size of less than 150 nm.

26. Composition according to any of claims 9 to 24, **characterised in that** it is an O/W micro-emulsion having an average particle size of less than 100 nm.

27. Moulded body for oral administration containing an active principle from the class of non-steroidal anti-inflammatory drugs in a composition according to any of claims 1 to 26 for administering the active principle.

28. Moulded body according to claim 27, **characterised in that** it contains a biopolymer.

29. Moulded body according to claim 27, **characterised in that** the biopolymer is gelatin.

30. Composition containing an active principle from the class of non-steroidal anti-inflammatory drugs according to any of claims 1 to 26, for topical administration.

31. Composition containing an active principle from the class of non-steroidal anti-inflammatory drugs according to any of claims 1 to 26, for cutaneous administration.

## Revendications

1. Composition sous forme d'un préconcentrat d'une microémulsion, contenant
(a) un mélange constitué d'un triglycéride et d'un acide gras oméga-9 et/ou d'un acide gras oméga-6 ; et
(b) un composant à activité de surface contenant un agent tensioactif,
(c) une substance active choisie dans la classe des inhibiteurs d'inflammation non stéroidiens, la substance active étant soluble dans (a) et/ou dans (b).

2. Composition selon la revendication 1, **caractérisée en ce qu'**on utilise en tant que triglycéride un triglycéride à chaîne moyenne.

3. Composition selon l'une des revendications 1 et 2, **caractérisée en ce qu'**on utilise en tant qu'agent tensioactif, un agent tensioactif du type polyoxyéthylène.

4. Composition selon l'une des revendications 1 à 3, **caractérisée en ce que** la substance active est choisie dans le groupe constitué des acides hétéroarylacétiques, arylacétiques, hétéroarylpropioniques et arylpropioniques et dans le groupe des inhibiteurs COX-2.

5. Composition selon l'une des revendications 1 à 3, **caractérisée en ce que** la substance active est choisie dans le groupe constitué de l'indométacine, du diclofénac, du naproxène, de l'ibuprofène, du dexibuprofène ainsi que du céléocoxib et du rofaecoxib.

6. Composition selon l'une des revendications 1 à 3, **caractérisée en ce que** la substance active est l'ibuprofène.

7. Composition selon l'une des revendications 1 à 3, **caractérisée en ce que** la substance active est le dexibuprofène.

8. Composition selon l'une des revendications 1 à 3, **caractérisée en ce que** la substance active est le naxoprène.

9. Composition sous forme d'une microémulsion pouvant être obtenue par mélange d'un préconcentrat d'une microémulsion selon l'une des revendications 1 à 8 avec de l'eau ou un milieu aqueux.

10. Composition selon l'une des revendications 1 à 9, contenant des composants supplémentaires qui n'appartiennent pas aux groupes de substances suivants :
- les (alkyl en C₁ à C₅) diéthers ou le tétrahydrofurfuryldiéther ou
- les éthers partiels de monooxy (alcane en C₂ à C₁₂)diols ou de polyoxy(alcane en C₂ à C₁₂)diols, de bas poids moléculaire ;
- le 1,2-propylèneglycol ;
- les alcanols inférieurs ;
- les produits d'estérification d'acides polycarboxyliques contenant 2 à 10, en particulier 3 à 5, groupes carboxyle avec des alcools en C₁ à C₁₀ ; et
- les produits d'estérification de polyols contenant 2 à 10, en particulier 3 à 5, groupes carboxyle avec des acides carboxyliques en C₂ à C₁₁.

11. Composition selon l'une des revendications 2 à 10, **caractérisée en ce que** les restes d'acide gras du triglycéride à chaîne moyenne présentent 4 à 18 atomes de carbone.

12. Composition selon l'une des revendications 2 à 10, **caractérisée en ce que** les restes d'acide gras du triglycéride à chaîne moyenne présentent 6 à 18 atomes de carbone.

13. Composition selon la revendication 11 ou 12, **caractérisée en ce que** le triglycéride à chaîne moyenne est un triglycéride d'acide caprylique/acide caprique.

14. Composition selon l'une des revendications 1 à 13, **caractérisée en ce que** l'acide gras oméga-9 et/ou l'acide gras oméga-6 présente(nt) 12 à 24 atomes de carbone.

15. Composition selon l'une des revendications 1 à 13, **caractérisée en ce que** l'acide gras oméga-9 et/ou l'acide gras oméga-6 présente(nt) 16 à 24 atomes de carbone.

16. Composition selon l'une des revendications 1 à 13, **caractérisée en ce que** l'acide gras oméga-9 et/ou l'acide gras oméga-6 présente(nt) 18 à 22 atomes de carbone.

17. Composition selon l'une des revendications 1 à 16, **caractérisée en ce que** l'acide gras oméga-9 est l'acide oléique.

18. Composition selon l'une des revendications 1 à 17, **caractérisée en ce que** l'acide gras oméga-6 est l'acide linoléique.

19. Composition selon l'une des revendications 1 à 18, **caractérisée en ce qu'**elle contient en tant que composant (a) un mélange d'un triglycéride de l'acide caprique/acide caprylique, d'acide oléique et/ou d'acide linoléique.

20. Composition selon l'une des revendications 1 à 19, **caractérisée en ce que** le rapport des quantités des acides gras oméga-9 et/ou oméga-6 au triglycéride à chaîne moyenne est de 1:1 à 1:200.

21. Composition selon l'une des revendications 1 à 19, **caractérisée en ce que** le rapport des quantités des acides gras oméga-9 et/ou oméga-6 au triglycéride à chaîne moyenne est de 1:2 à 1:20.

22. Composition selon l'une des revendications 1 à 20, **caractérisée en ce que** le composant à activité de surface (b) contient un ester d'acide gras et de polyoxyéthylène sorbitane, une huile végétale naturelle ou hydrogénée polyoxyéthylène-glycolysée ou des mélanges de ceux-ci.

23. Composition selon l'une des revendications 1 à 8 et 10 à 22, **caractérisée en ce que** le composant (a) est présent en une quantité de 5 à 60% en poids par rapport au poids total de la composition.

24. Composition selon l'une des revendications 1 à 8 et 10 à 23, **caractérisée en ce que** le composant à activité de surface (b) est présent en une quantité de 20 à 90% en poids par rapport au poids total de la composition.

25. Composition selon l'une des revendications 9 à 24, **caractérisée en ce qu'**il s'agit d'une microémulsion H/E présentant une grosseur moyenne des particules inférieure à 150 nm.

26. Composition selon l'une des revendications 9 à-24, **caractérisée en ce qu'**il s'agit d'une microémulsion H/E présentant une grosseur moyenne des particules inférieure à 100 nm.

27. Corps façonné pour l'administration par voie orale, contenant une substance active de la classe des inhibiteurs d'inflammation non stéroidiens dans une composition selon l'une des revendications 1 à 26 pour l'administration de la substance active.

28. Corps façonné selon la revendication 27, **caractérisé en ce qu'**il contient un biopolymère.

29. Corps façonné selon la revendication 27, **caractérisé en ce que** le biopolymère est de la gélatine.

30. Composition contenant une substance active de la classe des inhibiteurs d'inflammation non stéroidiens, selon l'une des revendications 1 à 26, pour une administration topique.

31. Composition contenant une substance active de la classe des inhibiteurs d'inflammation non stéroïdiens, selon l'une des revendications 1 à 26, pour une administration cutanée.
